# EUROPEAN PATENT APPLICATION

(11) **EP 1 516 873 A1**
(43) Date of publication of application: **23.03.2005**
(21) Application number: 03447231.6
(22) Date of filing: 22.09.2003
(51) Int. Cl.: C07D 213/80, C07D 213/69, A61K 31/4412, A61P 31/18

(54) **2-pyridinone derivatives, having HIV inhibiting properties**

(71) Applicant: Facultés Universitaires Notre-Dame de la Paix, 5000 Namur (BE); UNIVERSITE LIBRE DE BRUXELLES, B-1050 Bruxelles (BE)
(72) Inventor: Le Van, Kiet, 1050 Brussels (BE); Georges, Benoit, 1348 Louvain-La-Neuve (BE); Hevesi, Laszlo, 5100 Jambes (BE); Cauvin, Christine, 5101 Erpent (BE); Boland, Sandro, 4210 Burdinne (BE); Durant, Francois, 5000 Namur (BE); Demonte, Dominique, 7011 Ghlin (BE); Van Lint, Carine, 1150 Brussels (BE); Burny, Arsène, 5030 Gembloux (BE); Bollen, Alex, 1701 Itterbeek (BE); Jacquet, Alain Service de génétique appliquée, 6041 Gosselies (BE); De Walque, Stéphane, 1350 Folx-les-Caves (BE)
(74) Representative: Van Malderen, Joelle

(57) **Abstract**

The present invention relates to 2-Pyridinone derivatives, more specifically 5-ethyl-6-methyl-2-pyridinone derivatives, according to general formula I that inhibit human immunodeficiency virus type 1 (HIV-1) replication and are therefore of interest in the treatment of Acquired Immune Deficiency Syndrome (AIDS). The present invention further relates to the synthesis of said compounds and their use, with or without other pharmaceutical agents, in the treatment of AIDS and viral infections by HIV-1.

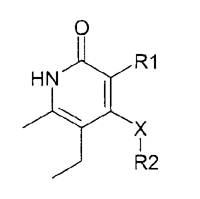

## Description

### Field of the invention

The present invention relates to 2-pyridinone derivatives, in particular 5-ethyl-6-methyl-2-pyridinone derivatives, that inhibit HIV, especially human immunodeficiency virus type 1 (HIV-1) replication and are therefore of interest in the treatment of Acquired Immune Deficiency Syndrome (AIDS). The present invention further relates to the synthesis of said compounds and their use, whether or not in combination with other pharmaceutical and/or therapeutic agents, in the treatment of viral infectious diseases like AIDS, especially viral infections by HIV-1.

### Background of the invention

Human Immunodeficiency Virus (HIV) is the causative agent of AIDS. Two main forms of this virus (HIV-1 and HIV-2) have been identified. HIV-O is a subtype of HIV-1. HIV-1, HIV-2 and HIV-O are all causative agents of AIDS, of which HIV-1 is the most common one. As a retrovirus from the lentivirus family, HIV has its genome in the form of single-stranded RNA.

An essential step of HIV lifecycle is therefore the reverse transcription of this single stranded RNA into double-stranded DNA. This process is catalyzed by a virally encoded enzyme known as reverse transcriptase. Numerous reverse transcriptase inhibitors have been used as antiretroviral agents. Most of them can be classified either as nucleoside reverse transcriptase inhibitors (NRTIs), also known as nucleoside analogues, or as non-nucleoside reverse transcriptase inhibitors (NNRTIs) that bind at an allosteric site (referred to as "TIBO site") some 10 Å from the catalytic site. Most NNRTIs display marked selectivity for HIV-1 inhibition.

### State of the art

European patent application EP0462800 describes a first series of pyridinone derivatives and their use in the treatment of HIV-related diseases.

European patent application EP0462808 discloses a series of pyridinone derivatives that are structurally related to those of EP0462800 and also find their use in the treatment of HIV-related diseases.

European patent application EP0481802 describes the preparation of 2-pyridinones and 2-pyridinethiones and their use in the treatment of HIV-related diseases.

International patent application WO97/05113 discloses the preparation of 4-aryl-thio-pyridinones and their use in the treatment of HIV-related diseases.

International patent application WO02/08226 discloses tricyclic 2-pyridinone compounds which are useful as inhibitors of HIV reverse transcriptase.

Published US patent application US2003125340 discloses 3-(Amino-or aminoalkyl) pyridinone derivatives and their use for the treatment of HIV related diseases.

International patent application WO99/55676 discloses the preparation of 3-amino- and 3-aminoalkyl-pyridinone and pyridinethione derivatives and their use in the treatment of HIV-related diseases.

International patent application WO02/24650 and European patent application EP 1 318 995 disclose another series of pyridinone and pyridinethione derivatives displaying HIV inhibiting properties.

The present invention provides still further antiviral agents with excellent activity against HIV-1 infections.

### Aims of the invention

The present invention aims to provide new antiviral agents that are able to prevent, inhibit and/or suppress viral infections and that show especially improved inhibitory action towards Human Immunodeficiency Virus type 1 (HIV-1) replication (reversible or irreversible inhibitors active against wild-type and mutant strains).

In particular, the present invention aims to provide such compounds which are non-nucleoside reverse transcriptase inhibitors (NNRTIs), able to block HIV-1 replication, and which do not require metabolic activation (e.g. phosphorylation) to be active.

A further aim of the present invention is to provide such compounds, which can be used in the prevention, suppression and/or the treatment of viral infections, either as pure compounds, as pharmaceutically acceptable salts or as prodrug thereof and/or as ingredient of a pharmaceutical composition, possibly in combination with other antiviral active agents and/or immunomodulators.

A last aim of the present invention is to provide methods of synthesis for such compounds and to provide compounds obtainable by said methods.

### Summary of the invention

One aspect of the invention concerns the antiviral compounds of claim 1. In particular, the present invention is related to new non-nucleoside reverse transcriptase inhibitor compounds, which display HIV-1 inhibitory properties, having the general formula I: 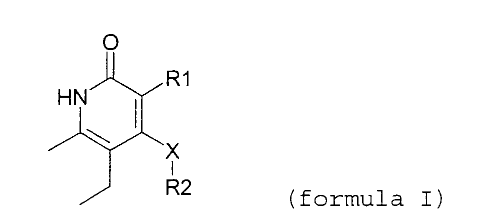 wherein
**X =** O, S, NH, C=O, (CₙH₂ₙ), (CₙH₂ₙ)O, O(CₙH₂ₙ), (CₙH₂ₙ)S, S(CₙH₂ₙ)with n = 1-4 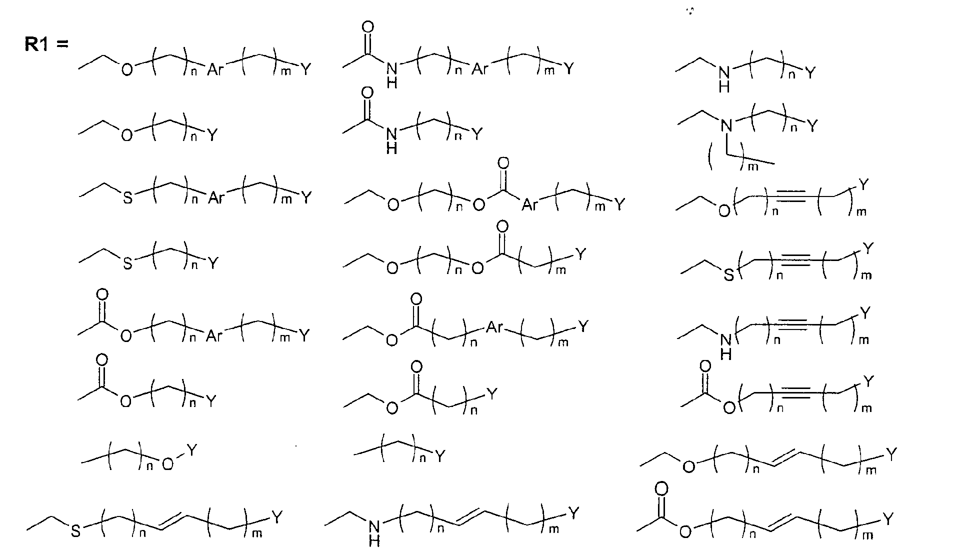 with n, m = 0 - 8
- **Ar =**: Aromatic ring selected from : phenyl, pyridyl, thiazolyl, furanyl, thiophenyl, benzofuranyl, benzothiophenyl, benzothiazolyl, imidazolyl, indolyl,
each optionally substituted with up to 4 substituants selected from : halo, hydroxy, C₁₋₄ alkyl, C₁₋₄ alkoxy, C₁₋₄ hydroxyalkyl, C₁₋₄ alkylamino, amino, C₁₋₄ aminoalkyl, C₁₋₄ alkylcarbonyl, C₁₋₄ dialkylamino, azido
- **Y =**: H, halo, alkylamino, dialkylamino, nitrile, hydroxy, C₁₋₆alkyloxycarbonyl, C₁₋₆alkylcarbonyloxy, C₅₋₇ cycloalkyl optionally substituted with up to 4 substituants selected from : halo, hydroxy, C₁₋₄ alkyl, C₁₋₄ alkoxy, C₁₋₄ hydroxyalkyl, C₁₋₄ alkylamino, amino, C₁₋₄ aminoalkyl, C₁₋₄ alkylcarbonyl, C₁₋₄ dialkylamino, azido, nitrile; or Y can be :

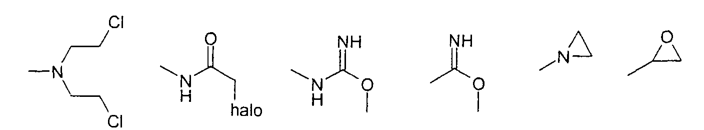

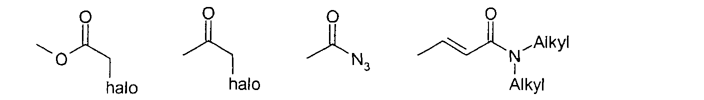

- **R2 =**: C₇₋₉ cycloalkyl;
C₅₋₈ cycloalkyl substituted with up to 4 substituants;
C₅₋₈cycloalkenyl optionally substituted with up to 4 substituants;
C₅₋₈aliphatic heterocycle optionally substituted with up to 4 substituants;
C₆₋₉bridged cycloalkyl optionally substituted with up to 4 substituants;
C₆₋₉bridged cycloalkenyl optionally substituted with up to 4 substituants;
substituants selected from :
halo, hydroxy, C₁₋₄ alkyl, C₁₋₄ alkoxy, C₁₋₄ hydroxyalkyl, C₁₋₄ alkylamino, amino, C₁₋₄ aminoalkyl, C₁₋₄ alkylcarbonyl, C₁₋₄ dialkylamino, azido, CN; 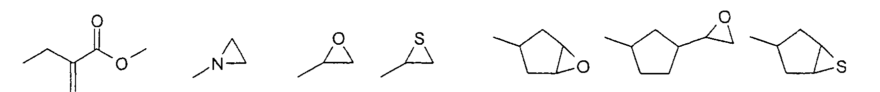 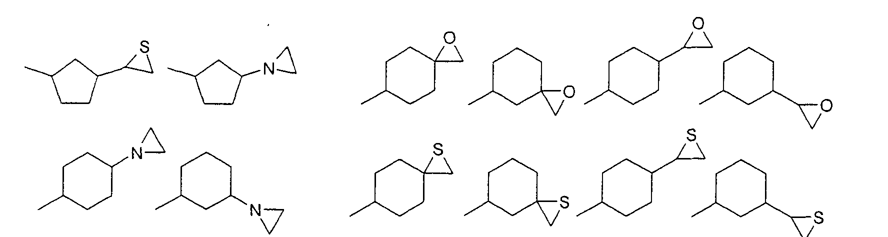 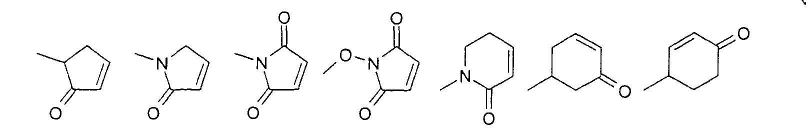 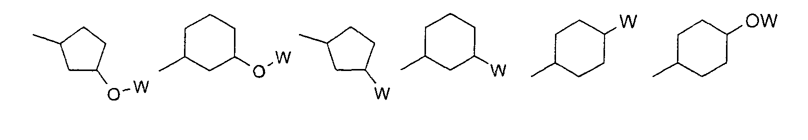 Or R2 can be :

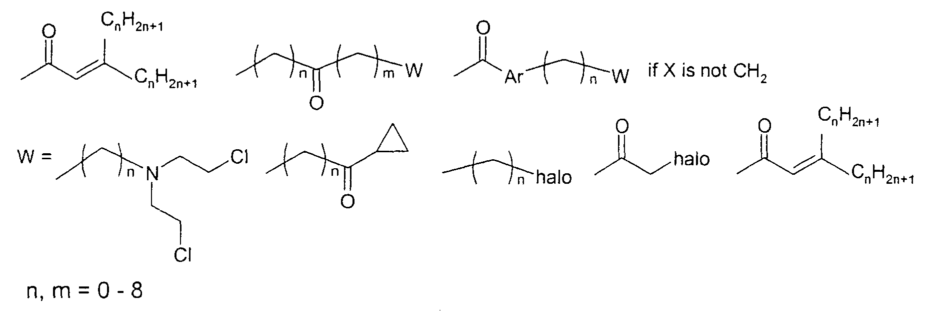

The present invention is also related to pharmaceutically acceptable salts or prodrugs of said compounds as well as to a pharmaceutical composition comprising at least one of the different compounds according to the invention (in pure form and/or as acceptable salt and/or as prodrug) and further an adequate pharmaceutical carrier and/or diluent. The compounds according to the invention may be used in combination with any other suitable (known or yet unknown) antiviral compounds, anti-infective agents, immunomodulators, antibiotics and/or vaccines.

Said pharmaceutical composition can find advantageous and efficient use in the prevention, treatment and/or the suppression of viral infections by Human Immunodeficiency Virus type 1 (HIV-1).

Another aspect of the present invention is related to the use of any of the compounds according to the invention (in pure form and/or as salt and/or as prodrug) or the pharmaceutical composition according to the invention as a medicament and/or for the manufacture of a medicament to treat, suppress and/or prevent viral infections induced by Human Immunodeficiency Virus type 1 (HIV-1).

A final aspect of the present invention is related to the preparation method of said compounds as described in detail hereafter.

The following examples and specific embodiments are intended for illustration purposes only, and should not be construed as limiting the scope of the invention in any way.

### Brief description of the Figures

The Figure 1 represents the synthesis of ethyl 4-[(3,5-dimethylcyclohexyl)oxy]-5-ethyl-6-methyl-pyridine-2(1H)-one-3-carboxylate (compound **Z37**).

The Figure 2A and 2B represent respectively the X-ray structure of compound **Z37A** and **Z37inv.**

The Figure 3 represents the synthesis of 4-(cycloheptyloxy)-3-(hydroxymethyl)-5-ethyl-6-methylpyridin-2(1H)-one (compound **Z32**)**.**

The Figure 4 represents the synthesis of [4-(cycloheptyloxy)-5-ethyl-6-methyl-2-oxo-1,2-dihydropyridin-3-yl]methyl chloroacetate (compound **Z33**).

The Figure 5 represents the synthesis of 2-(dimethylamino)ethyl 4-[(3,5-dimethylcyclohexyl)oxy]-5-ethyl-6-methyl-pyridine-2(1H)-one-3-carboxylate (compound **Z53**)**.**

The Figure 6 represents the synthesis of ethyl 5-ethyl-6-methyl-4-[(3-methylbut-2-enoyl)oxy]-2-oxo-1,2-dihydropyridine-3-carboxylate (compound **M18**).

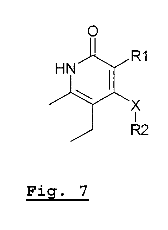

The Figure 7 represents general formula I.

### Detailed description of the invention

Compounds of general formula I (see above) that are described in the present invention behave either as reversible reverse transcriptase inhibitors or as irreversible reverse transcriptase inhibitors. The following two-step mechanism is thought to be involved in irreversible inhibition:
1. reversible binding to the allosteric site (TIBO site) of HIV-1 reverse transcriptase, and
2. formation of a covalent bond with a reactive aminoacid of the TIBO site, leading to irreversible inhibition

Of particular interest are compounds of formula I with a specific substitution in position 4 of the pyridinone ring. Such compounds show an excellent antiviral activity against HIV-1. A particular example hereof is for instance compound **Z37**, which bears a 3,5-dimethylcyclohexyl moiety as R2 (see general formula above).

Some of the compounds according to the present invention were found to exhibit an excellent antiviral activity against HIV-1 mutant strains that are resistant to one or more antiviral agents active against HIV-1 such as commonly applied NNRTIs like Nevirapine.

### Examples

### Example 1: Synthesis of ethyl 4-[(3,5-dimethylcyclohexyl) oxy)-5-ethyl-6-methyl-pyridine-2(1H)-one-3-carboxylate (compound Z37)

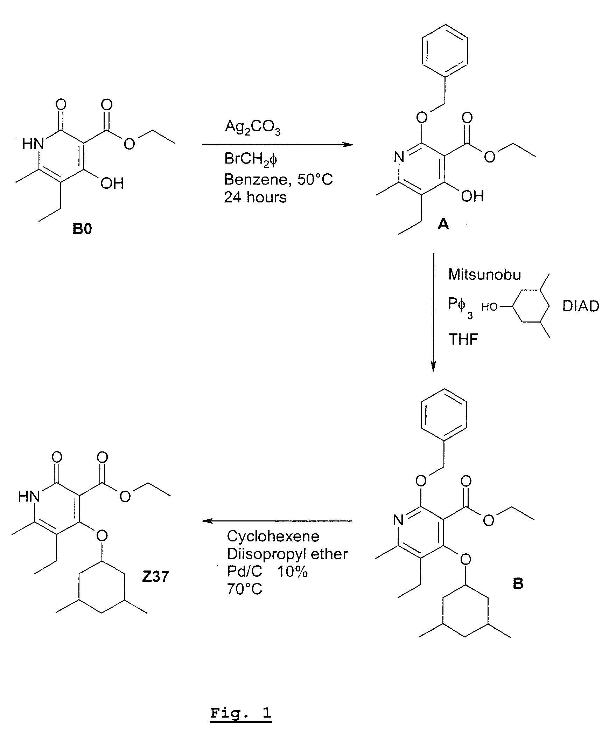

Compound **Z37** which corresponds to formula II 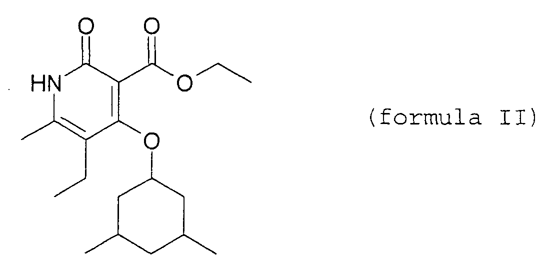 was synthesized, following a three-step protocol, as described below and as illustrated in Figure 1.

### Step 1

ethyl 4-hydroxy 5-ethyl-6-methyl-pyridine-2(1H)-one-3-carboxylate (B0) was synthesized as described by E. Bisagni and al. (J.Med.Chem. 1995, 38, 4679-4686). Then, benzyl bromide (1.8g, 10.5 mmol) was added to a stirred suspension of silver carbonate (1.41g, 5.1mmol) and B0 (2.25g, 10mmol). The mixture was heated (50°C) overnight then cooled and filtered over celite 521 (Aldrich). The solvent was evaporated and the crude product purified using a silica gel column (e.g. a 60Å/0.040-0.063mm ROCC column; eluent: pentane/dichloromethane, 70/30 v/v%), to give intermediate **A** (2.6g, 83% yield).

### Step 2

In a second step, Diisopropyl azodicarboxylate (DIAD) (0.804g, 4 mmol) was added drop wise at room temperature to a solution of intermediate A (0.63 g, 2 mmol), triphenylphosphine (PΦ₃) (1.048g, 4 mmol) and 3,5-dimethylcyclohexanol (0.512g, 4 mmol) in THF (20ml). After stirring overnight, the THF was evaporated and the residue was suspended in a mixture of hexane and diethyl ether (50:50 v/v%). The precipitate was filtered off and the organic layer was evaporated. The residue obtained was purified using a silica gel column (e.g. a 60Å/0.040-0.063mm ROCC column; eluent: pentane/dichloromethane, 50/50 v/v%), to give intermediate **B** (0.595 g, 70% yield).

### Step 3

In a third step, Pd/C 10% (w/w%) (0.160g) was added to a solution of intermediate **B** (0.360g, 0.89 mmol) in cyclohexane (4ml) and diisopropyl ether (12 ml). The mixture was heated overnight at 70°C. The precipitate was then filtered off and the organic solvents were evaporated. The product was purified with a silica gel column (e.g. a 60Å/0.040-0.063mm ROCC column; eluent: dichloromethane/ethanol, 95/05 v/v%) to give product **Z37** as a mixture of stereoisomers (0.238 g, 80% yield, mp (melting point) for the mixture of stereoisomers = 108°C).

The major isomer of the mixture, **Z37A** (70%) was purified by chiral HPLC (e.g. using a DAICEL chiralpak AD 4,6/250mm column; eluent: hexane/isopropanol 95/05 v/v%). (mp= 122°C).

A nuclear magnetic resonance (NMR) 1H profile was obtained using an Ex 90 FT NMR spectrometer (Jeol) and gave the following information for compound **Z37A**:
NMR 1H for **Z37A**: δ 13 (s, 1H), 4.7 (m, 1H), 4.4 (q, 2H), 2.4 (q, 2H), 2.25 (s, 3H), 2.15-1.6 (m, 8H), 1.35 (t, 3H), 1.0 (t,3H), 0.85 (d, 6H)

A diastereoisomeric form of Z37A (**Z37inv**) was obtained by a stereoselective double Mitsunobu reaction (mp= 158°C) (David L. Hugues 1992, "The mitsunobu reaction", Organic Reaction, 42, 335). Its antiviral activity was found to be higher than the activity observed for either **Z37A** or the mixture of stereoisomers (see infra).

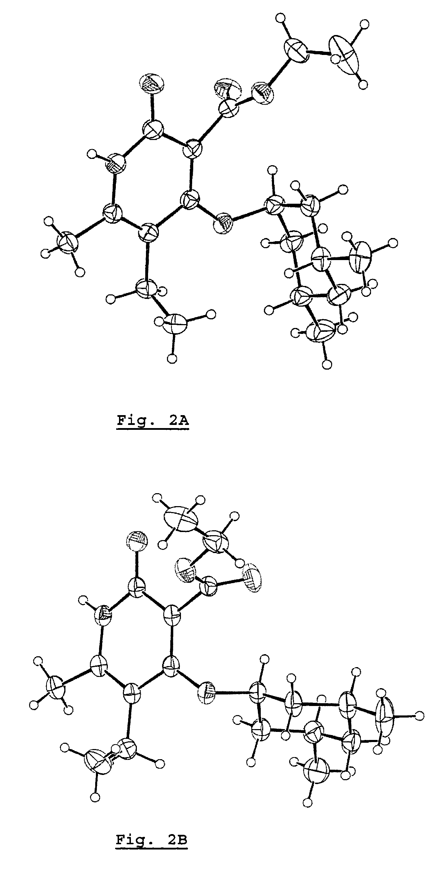

The stereochemistry of compounds **Z37A** and **Z37inv** was checked by X-Ray diffraction using a Enraf-Nonius CAD-4 apparatus (Brucker). The X-Ray diffraction structures of both compounds are given in Figures 2 A and B respectively. The crystal data of both compounds, as well as the specific data collection information and applied refinement conditions, are summarized in Table 1.

**Table 1:**

| Crystal data, data collection and refinement information for compounds **Z37A** and **Z37inv** (symbols used are standard IUPAC symbols well known in the art) | | |
|---|---|---|
| | **Z37A** | **Z37inv** |
| **Crystal Data** | | |
| Formula | C₁₉H₂₉NO₄ | C₁₉H₂₉NO₄ |
| MW | 335.43 | 335.43 |
| System, space group | Triclinic, P-1 | Monoclinic, P21/c |
| a (Å) | 8.518(1) | 13.488(3) |
| b (Å) | 9.220(2) | 9.064(2) |
| c (Å) | 13.436(1) | 16.584 (1) |
| α (°) | 88.906(6) | 90.0 |
| β (°) | 82.053(4) | 110.617(16) |
| γ (°) | 66.369(7) | 90.0 |
| V (Å³) | 956.7 (2) | 1897.6(6) |
| Z | 2 | 4 |
| Dₓ (Mg m⁻³) | 1.164 | 1.174 |
| Radiation | Cu K_{α} | Cu K_{α} |
| µ (mm⁻¹) | 0.651 | 0.066 |
| T (K) | 293(2) | 293(2) |
| Crystal | Platelet, colourless | Platelet, colourless |
| Crystal size | 0.34 x 0.30 x 0.13 | 0.45 x 0.34 x 0.25 |

| **Data collection** | | |
|---|---|---|
| Diffractometer | Enraf-Nonius CAD-4 | Enraf-Nonius CAD-4 |
| Scan | θ/2θ | θ/2θ |
| Absorption correction | Analytical Tₘᵢₙ=0.809, Tₘₐₓ=0.920 | None |
| Measured reflections | 4012 | 4075 |
| Independent reflections | 3764 | 3907 |
| Reflections with I > 2 σ (I) | 3067 | 3223 |
| Rᵢₙₜ | 0.0137 | 0.0967 |
| θₘₐₓ | 71.97 | 74.98 |
| h | -9 -> 10 | 0 -> 16 |
| k | 0 -> 11 | 0 -> 11 |
| l | -16 -> 16 | -20 -> 19 |

**Table 1: continuation**

| **Refinement** | | |
|---|---|---|
| Refinement on | F² | F² |
| R [F² > 2 σ (F²)] | 0.0526 | 0.0825 |
| wR (F²) | 0.1759 | 0.2150 |
| S | 1.462 | 1.689 |
| Number of reflections | 3764 | 3907 |
| Number of parameters | 223 | 221 |
| (Δ/σ)ₘₐₓ | 0.013 | 0.001 |
| Δρₘₐₓ | 0.289 | 0.421 |
| Δρₘᵢₙ | -0.299 | -0.335 |

### Example 2: Synthesis of 4-(cycloheptyloxy)-3-(hydroxymethyl)-5-ethyl-6-methylpyridin-2(1H)-one (compound Z32)

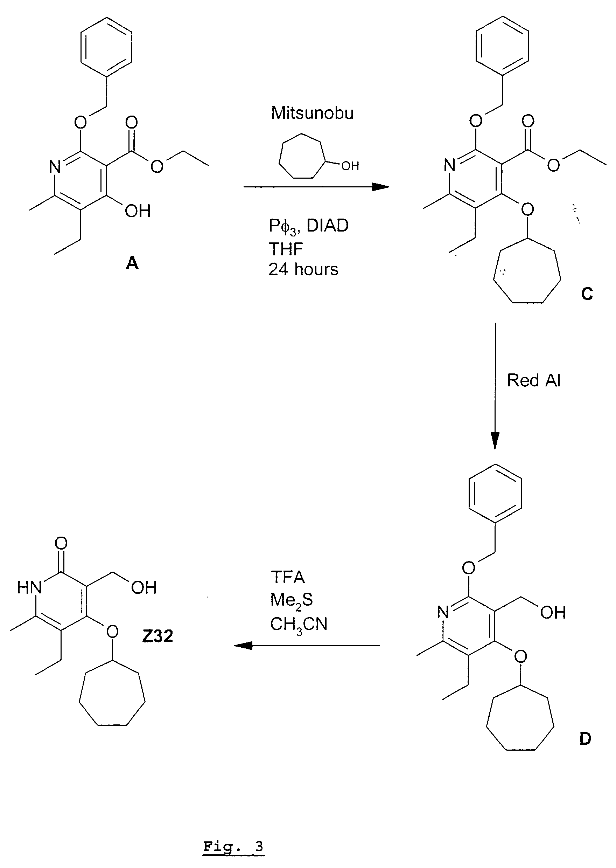

Compound **Z32** which corresponds to formula III 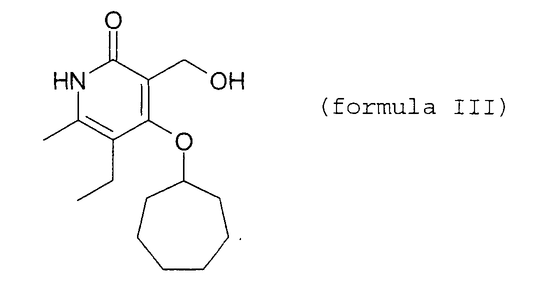 was synthesized, following a three-step protocol, as described below and as illustrated in Figure 3.

### Step 1

In a first step, Diisopropyl azodicarboxylate (DIAD) (0.804g, 4 mmol) was added drop wise at room temperature to a solution of above-described intermediate A (0.63 g, 2 mmol), triphenylphosphine (PΦ₃) (1.052g, 4 mmol) and cycloheptanol (0.456g, 4 mmol) in THF (20ml). After stirring overnight, the THF was evaporated and the residue was suspended in a mixture of hexane and diethyl ether (50:50 v/v%). The precipitate was filtered off and the organic layer was evaporated. The residue obtained was purified using a silica gel column (e.g. a 60Å/0.040-0.063mm ROCC column; eluent:pentane/dichloromethane, 50/50 v/v%), to give intermediate C (0.575 g, 70% yield).

### Step 2

In a second step, Red-A1 (2.3 ml, 7.6 mmol) was suspended in benzene (10 ml). Intermediate C (1.77g, 4.3 mmol) was added to this solution, at 0°C. The mixture was heated at 75°C for 2 hours and then cooled again at 0°C. After addition of a solution of 20% sulphuric acid, the aqueous layer was extracted with dichloromethane. The organic extracts were collected and washed with brine.

The crude product was chromatographed on silica gel column (e.g. a 60Å/0.040-0.063mm ROCC column; eluent: dichloromethane/pentane, 50/50 v/v%) to afford intermediate **D** (1.507 g, 95% yield).

### Step 3

In a third step, Intermediate **D** (1.5g, 4 mmol) was then dissolved in a mixture of acetonitrile (10 ml) and dimethyl sulfide (2 ml). Trifluoroacetic acid (lml) was then added to this mixture, at 0°C. After stirring at room temperature for 3 hours, the solvents were evaporated. The residue was dissolved in dichloromethane and washed with a solution of saturated NaHCO₃.

After drying with MgSO₄ and evaporation of the solvent, recrystallisation from ethyl acetate/hexane gave pure product **Z32** as white crystals (mp = 146°C)

A nuclear magnetic resonance (NMR) 1H profile was obtained using an Ex 90 FT NMR spectrometer (Jeol) and gave the following information for compound **Z32**:
NMR 1H for **Z32:** δ 13 (s, 1H), 4.6 (s, 2H), 4.1 (m, 1H), 2.44 (q, 2H), 2.31 (s, 3H), 1.7 (m, 12H), 1.1 (t, 3H)

### Example 3: Synthesis of [4-(cycloheptyloxy)-5-ethyl-6-methyl-2-oxo-1,2-dihydropyridin-3-yl)methyl chloroacetate (compound Z33)

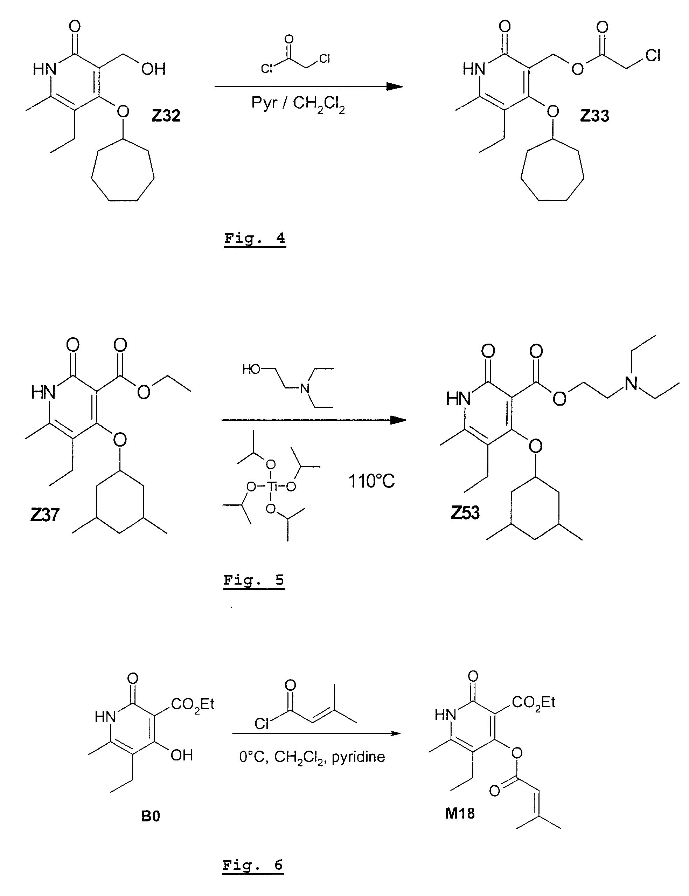

Compound **Z33** which corresponds to formula IV 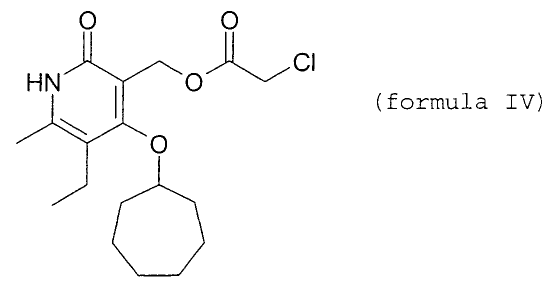 was synthesized from compound **Z32** as described below and as illustrated in Figure 4. Compound **Z32** (165 mg, 0.6 mmol) was dissolved in dichloromethane (2ml) and pyridine (50µl). Chloroacetyl chloride (50µl) was added to this mixture cooled at 0°C. After stirring for 3 hours at 0°C, HCl (1ml, 1N) and dichloromethane (10ml) were added to this solution.

The organic layer was washed with a saturated NaCl solution and the crude product purified using a silica gel column (eluent: MeOH/CH₂Cl₂, 1/9) to give the crystalline product **Z33** (0.160 g, Yield: 75%, mp = 123°C).

A nuclear magnetic resonance (NMR) 1H profile was obtained using an Ex 90 FT NMR spectrometer (Jeol) and gave the following information for compound **Z33**:
NMR 1H for **Z33**: δ.13 (s, 1H), 5.2 (s, 2H), 4.2 (m, 1H), 4.0 (s, 2H), 2.5 (m, 2H), 2.3 (s, 3H), 1.7 (m, 12H), 1.1 (m, 3H)

### Example 4: Synthesis of 2-(dimethylamino)ethyl 4-[(3,5-dimethylcyclohexyl)oxy]-5-ethyl-6-methyl-pyridine-2(1H)-one-3-carboxylate (compound Z53)

Compound **Z53** which corresponds to formula V 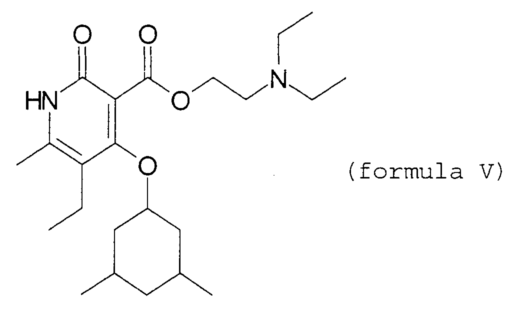 was synthesized from compound **Z37** as described below and as illustrated in Figure 5. To a solution of **Z37** (0.167g, 0.5 mmol) in N,N-diethylethanolamine (3ml) was added a catalytic amount of tetraisopropyl titanate (c.a. 30 mg). The mixture was stirred overnight at 110°C.

The solvent was then evaporated under vacuum and the residue was extracted with dichloromethane. The crude product was purified with a silica gel column (eluent: dichloromethane/ethanol, 90/10) to give product **Z53** (0.121 g, 60% yield, oil).

A nuclear magnetic resonance (NMR) 1H profile was obtained using an Ex 90 FT NMR spectrometer (Jeol) and gave the following information for compound **Z53**:
NMR 1H for **Z53**: δ.12.8 (s, 1H), 4.7 (m, 1H), 4.3 (t, 2H), 2.9-2.4 (m, 6H), 2.3 (s, 3H), 2.1-1.4 (m, 8H), 1.25-0.7 (m, 17H).

### Example 5: Synthesis of ethyl 5-ethyl-6-methyl-4-[(3-methylbut-2-enoyl)oxy]-2-oxo-1,2-dihydropyridine-3-carboxylate (compound M18)

Compound **M18** which corresponds to formula VI 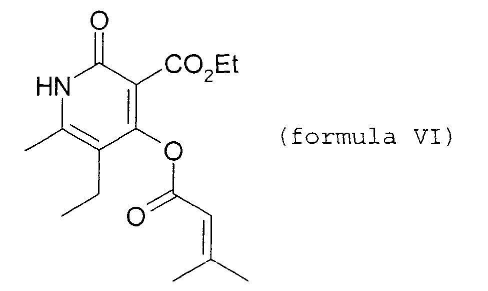 was synthesized as described below and as illustrated in Figure 6. Intermediate **B0** (0.338g, 1.5 mmol) was dissolved in dichloromethane (10 ml) and pyridine (1 ml). 3,3-dimethyl acryloyl chloride (0.360g, 3.0 mmol) was added to this solution at 0°C and the solution was stirred overnight. The solvents were then removed in vacuo. Purification by silica gel chromatography (e.g. a 60Å/0.040-0.063mm ROCC column; eluent: ethanol/dichloromethane, 98/02 v/v%) gave product **M18** (0.270g, 60% yield, mp = 166°C).

A nuclear magnetic resonance (NMR) 1H profile was obtained using an Ex 90 FT NMR spectrometer (Jeol) and gave the following information for compound **M18**:
NMR 1H for **M18:** δ 13 (s, 1H), 5.9 (s, 1H), 4.2 (q, 2H), 2.5 (m, 2H), 2.3 (s,3H), 2.2 (s, 3H), 2.0 (s, 3H), 1.3 (t, 3H), 1.1 (t, 3H)

Compounds **Z12, Z25, Z30, Z54** and **Z55** were synthesized in a similar way as compound **Z37**. The protocol is similar except for the alcohol used in the second step, which is 2-chlorocyclohexanol for **Z12**, cycloheptanol for **Z25**, 3-methylcyclohexanol for **Z30**, cyclooctanol for **Z54** and 4-ethylcyclohexanol for **Z55.**

Table 2 provides information on the structure and physico-chemical properties of specific compounds according to the invention, such as the nature of the side groups R1 and R2 and of the spacer X, the melting point of the compound and its molecular weight.

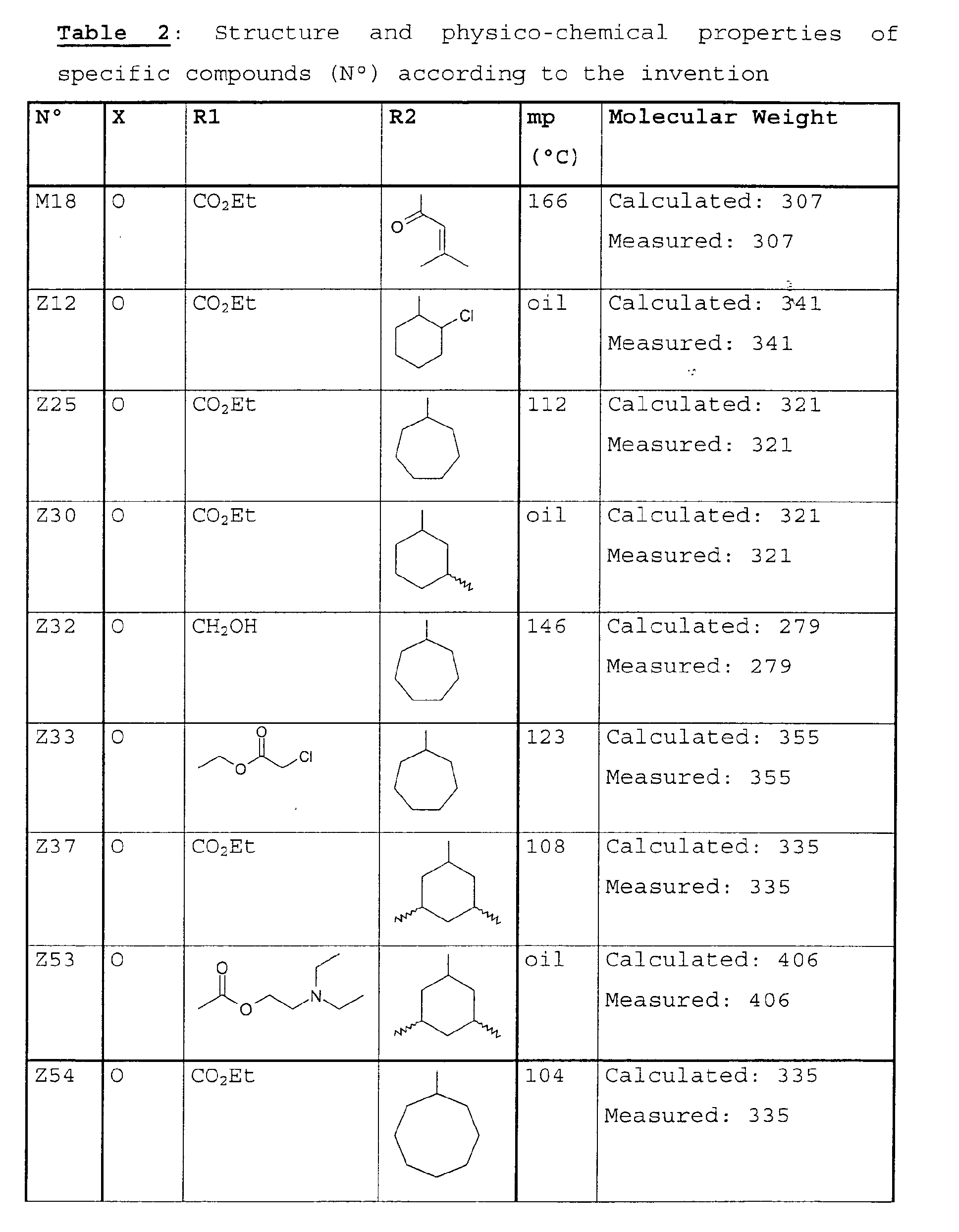

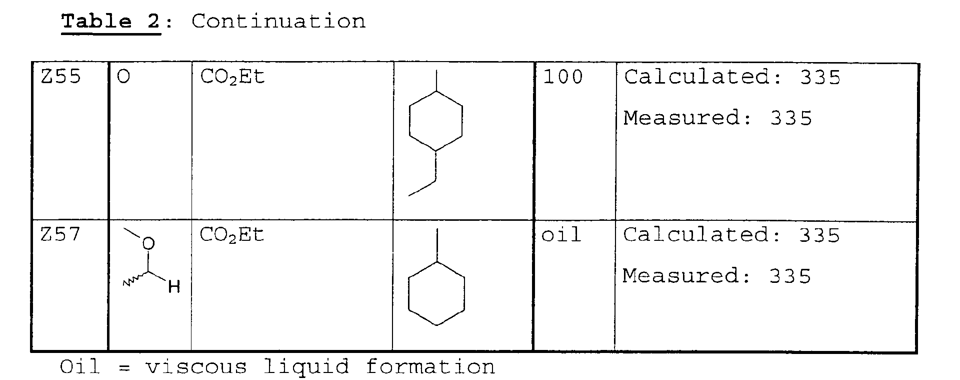

Examples 6 to 7 demonstrate that the compounds of the present invention are very efficient NNRTIs with HIV-1 inhibiting activity. This is illustrated via in vitro reverse transcriptase assays and via anti-HIV assays using P4 cell lines. The P4 cell line contains a LacZ gene under the transcriptional control of HIV-1 LTR elements.

For the *in vitro* inhibition studies of the HIV-1 reverse transcriptase activity, stock solutions of the compounds of the present invention were prepared in dimethyl sulfoxide at a final concentration of 10mM and kept at room temperature. Nevirapine was purchased from Boehringer Ingelheim.

For the antiviral and the cytotoxicity assays, the drugs were diluted in complete DMEM medium. In these experiments, drugs were diluted in triplicate wells in a 96-well plate in six, 5 fold serial dilutions.

### Example 6: Effect of the compounds according to the invention on the reverse transcriptase activity in vitro of HIV-1

### HIV-1 reverse transcriptase activity:

*In vitro* inhibition studies used a fixed-time assay for HIV-1 reverse transcriptase RNA dependent DNA polymerase activity. RT was purchased from VWR (ref CAL382129-500). One unit of RT corresponds to the amount of enzyme which incorporates one nanomole of [³H] TTP in 10 minutes at 37°C.

Assays were performed in a final volume of 50µl. The mixture contained 0.125 units of RT, 10 mM MgCl₂, 2mM DTT, 50mM Tris pH 8.3, 50mM KCl, 1µg/µl BSA, 0.01% triton X100, 20 µg/ml (0.4 A260/ml) poly(rC)-oligo(dG)_{12-18,} 1µCi [³H]dGTP and 1µl of the inhibitor (dissolved in dimethyl sulfoxide, DMSO). Reaction mixtures were incubated at 37°C for 10 min. The incorporation rate was determined by a standard trichloroacetic acid precipitation procedure (*adapted from Current protocols in molecular biology. Eds Wiley, MGH Harvard medical school*) and liquid scintillation counting using a Wallac scintillation counter.

### Production of Viral stocks:

Wurzburg Jurkat T cells (subclone JR) were transfected with 10 µg of the circularly permuted infectious molecular clone HIV_{PNL4-3} (Adachi et al., 1986. J. Virol, 59 (2), p284-291). Two days later, co-cultivation with SupT1 cells (a human T-cell lymphoma cell line) was initiated to facilitate rapid production of progeny virions. Production of virus was measured by using the *Innotest HIV Antigen mAb* p24 kit (Innogenetics). At the peak of production cultures were harvested and filtered. The amount of virus produced was measured by p24 ELISA experiments and the virus stocks were stored at -80°C until used.

Results for some of the compounds according to the invention are summarized in Table 3. The *in vitro* activity of the compounds, at a final concentration of 10 µM, on the reverse transcriptase (RT) activity of HIV-1 is derivable from the relative (%) reduction in RT activity. Hereby, the RT activity in the absence of any of the compounds is set at 100%. From table 3 it is evident that all of the compounds tested were able to reduce the *in vitro* RT activity by at least about 30 %. Most of the compounds tested reduced the activity by at least 50% to 60%. The most active compound, **Z37inv**, reduced the activity to about 6% only of the control. This activity is slightly better than the one of Nevirapine, a common NNRTI.

**Table 3:**

| *In vitro* residual RT activity after addition of some compounds (N°) belonging to the invention. Comparison with Nevirapine, a common RT inhibitor | |
|---|---|
| N° | Relative (%) RT activity *in vitro* (compound added at 10µM) |
| M18 | 70.8 |
| Z12 | 63.7 |
| Z25 | 34.6 |
| Z30 | 22.0 |
| Z32 | 44.0 |
| Z33 | 42.0 |
| Z37 | 8.3 |
| Z37A | 11.7 |
| Z37B | 8.0 |
| Z37inv | 6.7 |
| Z53 | 26.5 |
| Z54 | 19.4 |
| Z55 | 28.8 |
| Z57 | ND |
| Nevirapine | 8.6 |
| ND, not determined | |

### Example 7: Anti-HIV activity (EC₅₀ value expressed in µM) on P4 cells and cytotoxici ty (CC₅₀ value expressed in µM) of some of the compounds according to the invention P4 Cell line:

Anti-HIV activity and cytotoxicity of the compounds were tested on a P4 cell line. The P4 cell line (Clavel & Charneau, 1994. J. Virol., Vol. 68 p1179-1185) was provided by Dr. François Clavel (Unité de recherche antivirale de l'hôpital Xavier Bichat Paris: Inserm). These p4 cells were cultured in complete DMEM medium supplemented with 10% fetal bovine serum (FBS), 0.5 % of Penicillin/Streptomycin and G418 at 0.5 mg/ml. Exponentially growing cells were trypsinized, centrifuged and split twice weekly at 5.10⁴ cells/ml.

### Cytotoxicity of the compounds

The 50 % cytotoxic concentration (CC₅₀) was determined using a protocol adapted from Pauwels et al. (1988. J. Virol. Methods 20(4):309-21). Briefly, flat bottom 96-well plates were filled with 50 µl of complete medium containing 5.10³ P4 cells. 2 hours later 50 µl of drug solution were added to the cells. Drugs (dissolved in DMEM, see above) were diluted in six, 5-fold serial dilutions from stock solutions in triplicate wells of a 96-well plate. Cells and compounds were incubated at 37°C in growth medium for 3 days. Cell viability was determined by MTT assays using the *Roche Cell Proliferation KIT.* The absorbance (λ=570nm) was measured on a Benchmark™ Microplate Reader (Biorad) and compared with 12 cell control replicates (no drug added). Each assay was performed at least three times for a total of at least nine replicate wells. This method detects both cytostatic and cytolytic effects of drugs.

### Anti-HIV assay

The P4 cell line is a cell line of HIV-infectible Hela-CD4 cells that carry the bacterial lacZ gene under the control of the HIV-1 long terminal repeat (LTR). In this cell line, transcription of the LacZ gene is driven by the HIV-1 LTR. As such, the cytoplasmic accumulation of β-galactosidase is strictly dependent on the presence of the HIV transactivator Tat produced during the intracellular viral replication (Clavel & Charneau, 1994. J. Virol., Vol.68, p. 1179-1185). In other words, in this system, the expression level of the β-galactosidase gene is proportional to the viral replication.

In a particular embodiment, the p4 cell line was infected with a HIV strain having a mutant RT characterized by a Cystein for Tyrosine substitution (Y188C mutation). This mutant HIV cell line was produced as follows. In a first step, the Y188C mutation was introduced by site directed mutagenesis on the circularly permuted infectious molecular clone HIV_{PNL4-3}. In a second step, the resulting plasmid was transfected in Jurkat cells and the mutant viruses produced were purified as indicated above.

Briefly, in the anti-HiV assays used here 100 µl of P4 cells were plated in 96-well plate at a concentration of 0.4 10⁵ cells/ml and incubated at 37°C, 5 % CO₂. After 48h, the medium was removed and 100 µl of the different drugs dilutions were added to the cells. Four hours after the addition of the drugs all cells were infected with equal amount of cell-free virus, corresponding to 100 ng of HIV p24 antigen. After 48h of incubation at 37°C, 5% CO₂, β-galactosidase activity was measured using chlorophenol red- β -D-galactopyranoside assays.

The absorbance was measured on a Benchmark™ Microplate Reader (Biorad) (λ=570nm) and compared with 12 cell control replicates (no virus or drug added) and 12 virus control wells (no drug added). Each assay was performed a minimum of three times. The 50 % effective concentration (EC₅₀) was calculated from each dose response curve using the CurveExpert 1.3 software. As Nevirapine activity corresponds to its published values (10-100 nM), the data are consistent with other measures of viral replication.

The results of both tests are summarized in columns 2, 3 and 4 of Tables 4 and 5. From these tables it can be derived that the compounds according to the invention have good to excellent EC₅₀ values and are able to inhibit HIV-1 activity. Compound **Z37inv** gave the best result and is more active than Nevirapine. It displays a good selectivity index (SI) due to this high antiviral activity combined with a low cytotoxicity.

That some of the compounds, for instance compound **Z37**, are active on HIV-1 mutant strains resistant to Nevirapine is evident from Table 5.

From the above it is evident that the compounds according to the invention have very good anti-HIV activity. The demonstrated low cytotoxicity is a first indication that the compounds could be very useful in the treatment of HIV and especially HIV-1 infected individuals.

**Table 4:**

| *Ex vivo* anti-HIV activity (EC₅₀), cytotoxicity (CC₅₀) and SI (selectivity index = CC₅₀/EC₅₀) for some compounds according to the invention, the test being performed on a P4 cell line with a WT (wild-type) RT. Comparison with Nevirapine, a common RT inhibitor | | | |
|---|---|---|---|
| N° | EC₅₀ WT (µM) | CC₅₀ (µM) | SI |
| M18 | 5.82 | >100 | >17 |
| Z12 | 2.53 | >100 | >40 |
| Z25 | 0.48 | 81 | 168 |
| Z30 | 0.52 | >100 | >192 |
| Z32 | 1.16 | 55 | 47 |
| Z33 | 1.03 | 54 | 52 |
| Z37 | 0.043 | 58 | 1349 |
| Z37A | 0.240 | 63 | 263 |
| Z37B | 0.091 | 72 | 791 |
| Z37inv | 0.021 | 64 | >3047 |
| Z53 | >>4 | 33 | <8 |
| Z54 | 1.34 | 58 | 43 |
| Z55 | >>4 | 57 | <14 |
| Z57 | 1.82 | ND | ND |
| Nevirapine | 0.030 | >100 | >3333 |
| ND, not yet determined | | | |

**Table 5:**

| *Ex vivo* anti-HIV activity (EC₅₀), cytotoxicity (CC₅₀) and SI (selectivity index = CC_{50/}EC₅₀) for some compounds according to the invention, the test being performed on a P4 cell line with a mutant RT characterized by a Cystein for Tyrosine substitution at codon 188 (Cys188) in the RT. Comparison with Nevirapine, a common RT inhibitor | | | |
|---|---|---|---|
| N° | EC₅₀ Cys188RT (µM) | CC₅₀ (µM) | SI |
| M18 | 5.38 | >100 | >18 |
| Z37 | 0.006 | 58 | 9667 |
| Z37A | 0.039 | 63 | 1615 |
| Z37B | 0.025 | 72 | 2880 |
| Z37inv | 0.010 | 64 | 6400 |
| Z53 | >4 | 33 | <8 |
| Z54 | 0.120 | 58 | 483 |
| Z55 | 0.406 | 57 | 140 |
| Nevirapine | 2.77 | >100 | 36.1 |

From the above, it is evident that in contrast to Nevirapine, some compounds of the invention are active against Cys188 mutant strains.

The compounds according to the present invention could be administrated orally to humans in a dosage range of 1 to 100 mg/kg body weight in divided doses. It will be understood, however, that the specific dose level and frequency of dosage for any particular patient may vary and will depend upon a variety of factors including the activity of the compound employed, its metabolic stability and length of action, as well as the age, the weight and the general health of the patient at the time of the administration, the rate of excretion, the other drugs used, and the host undergoing therapy. It falls within the skills of an artisan to determine the concentration of drugs that should be used in HIV-1 treatment.

The compounds of the present invention can be used for the preparation of medicaments such as therapeutic compositions for the treatment of HIV-1 related diseases. The compounds can be used alone (in pure form, as salt or as prodrug), or as mixtures of several compounds, whether or not in combination with other compounds active against HIV-1 infections.

Such anti-viral agents include other NNRTIs such as Nevirapine, Efavirenz, Delavirdine, Capravirine and the like as well as NRTIs, protease inhibitors, fusion/binding inhibitors, integrase inhibitors, pyrophosphate analogue RT inhibitors and/or HIV vaccines. The above list is not exhaustive and may include any other anti-viral, anti-infective, antibiotic as well as any immunomodulator. The effect can be additive and/or synergistic.

The compounds according to the invention and mixtures thereof with any other therapeutic and/or pharmaceutical agent can be used in pharmaceutical compositions comprising an acceptable diluent and/or carrier. These are known to a skilled person.

Administration in the case of combinations can be together or consecutively whereby the interval can range from minutes to hours. It is evident that other applications than oral applications are possible, for instance in the case of combination with a therapeutic and/or prophylactic vaccine. It is further evident that the compounds according to the inventions can be applied under any form that does not preclude their activity, such forms including pills, liquids, powders, pastes and any other form or formulation known in the art.

### Example 8: Comparison of some compounds according to the invention with compounds disclosed in prior art and influence of the R2 type on the activity of the compound

Compounds according to the present invention were compared with compounds known in the art. Provided that any group that is linked to position 4 of the pyridinone ring is hereby referenced as "R2", and X is defined as the "spacer" between the two groups, the following can be concluded

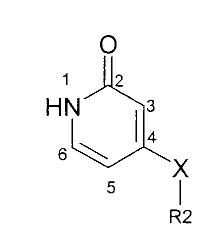

Compounds disclosed in patents EP 0 462 800 and EP 0 481 802 differ by their substitution in position 3 and differ by their substitution in position 4 (no spacer between the pyridinone and R2 in the compounds disclosed in EP 0462 800 and EP 0 481 802 whereas all compounds of the present invention have such a spacer).

The compounds disclosed in patent EP 0 462 808 differ by their substitution in position 3 (all possess a phtaloyl group on this position). They are further not substituted in position 4.

The compounds disclosed in WO97/05113 differ by their substitution in position 4. Only arylthio groups are considered, whereas compounds of the invention do not feature any arylthio group in position 4.

The compounds disclosed in WO99/55676 differ by their substitution in position 3. Only amino or alkylamino groups have been considered. None of these chemical functions is present in the invention.

Compounds disclosed in International patent application WO02/24650 are of the above the compounds most closely related with those of the present invention, the compounds having a spacer between the pyridinone ring and R2, the R2 groups at first sight maybe comparable. However, the specific compounds disclosed in WO02/24650 to have anti-HIV activity all feature an aryl substituent at position 4 of the pyridinone ring, unlike compounds of the present invention. No compound bearing a C₇₊ cycloalkyl or a substituted cycloalkyl in position 4 is disclosed and/or claimed in International patent application WO02/24650.

The following table 6 demonstrates the interest of for instance C₇₊ cycloalkyls or substituted cycloalkyls as evident from the EC50 value:

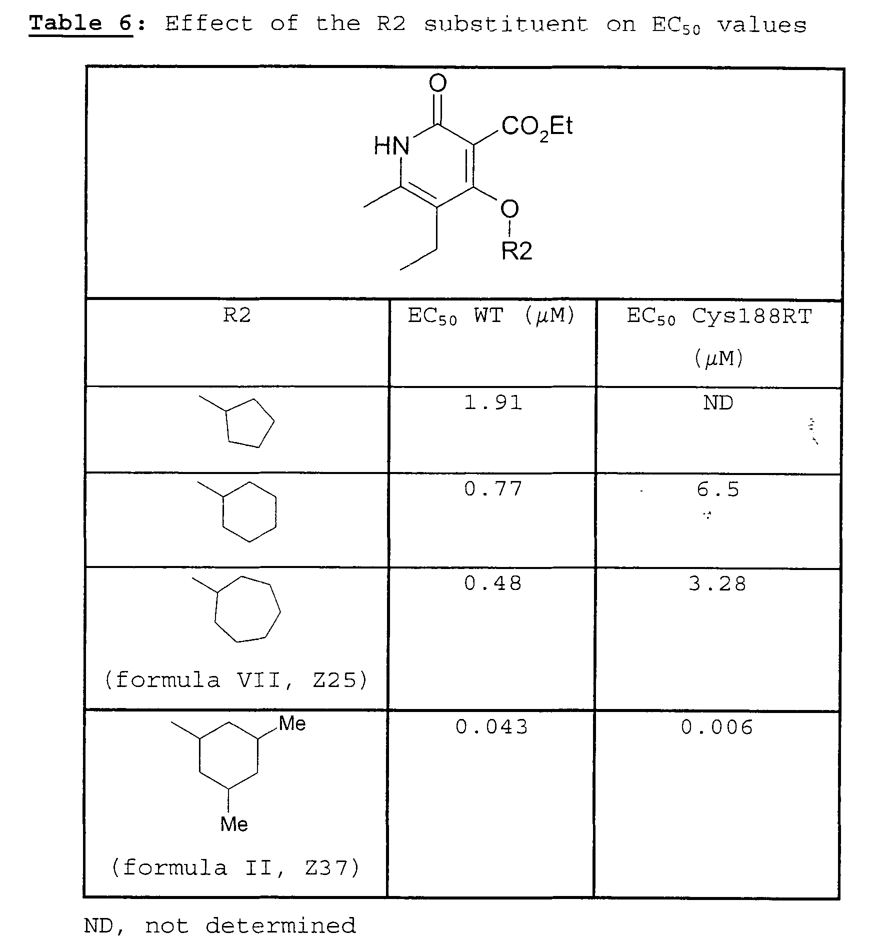

As shown in Table 6, replacement of a cyclopentyl or cyclohexyl by a cycloheptyl leads to an increase in antiviral potency of the compounds.

Replacement of a cyclohexyl by a m,m-dimethyl-cyclohexyl leads to a 16-fold increase in antiviral potency.

Furthermore, compounds **Z25** and **Z37** are more active on Cys188 mutant strains than the cyclohexyl derivative.

It goes beyond any doubt that the above examples are sufficient to demonstrate that the problem of providing alternative compounds active against HIV-1, with pronounced NNRTI activity, is solved by the compounds according to the invention which are novel and inventive.

Advantageously, compounds according to the invention can be active against HIV-1 strains that are resistant to NNRTIs currently used such as Nevirapine.

## Claims

1. A 5-ethyl-6-methyl-2-pyridinone derivative compound according to general formula I, 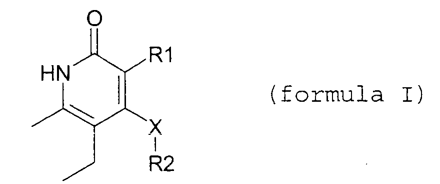 wherein
**X =** O, S, NH, C=O, (CₙH₂ₙ), (CₙH₂ₙ)O, O(CₙH₂ₙ), (CₙH₂ₙ)S, S(CₙH₂ₙ)with n = f-4 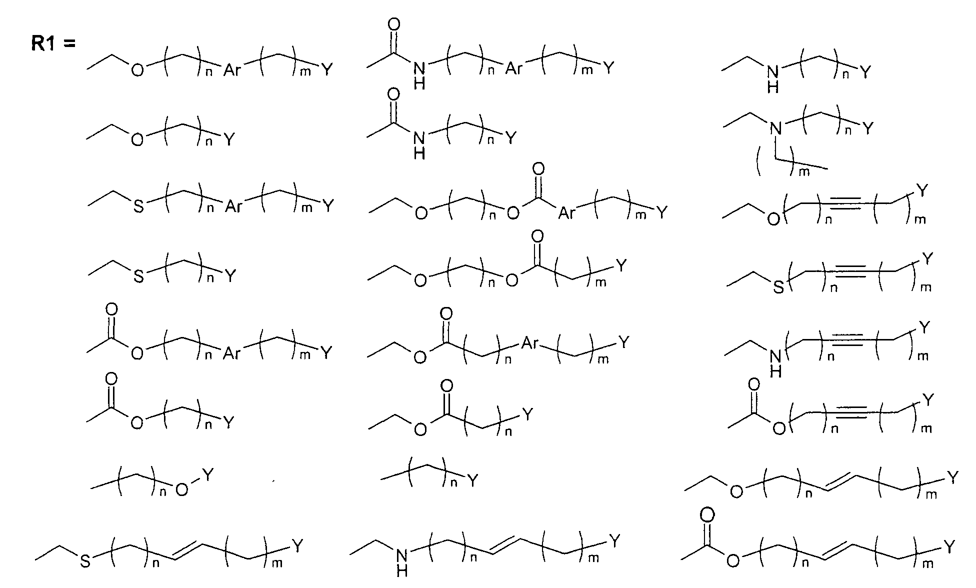
with n, m = 0 - 8
**Ar =** Aromatic ring selected from : phenyl, pyridyl, thiazolyl, furanyl, thiophenyl, benzofuranyl, benzothiophenyl, benzothiazolyl, imidazolyl, indolyl,
each optionally substituted with up to 4 substituants selected from :
halo, hydroxy, C₁₋₄ alkyl, C₁₋₄ alkoxy, C₁₋₄ hydroxyalkyl, C₁₋₄ alkylamino, amino, C₁₋₄ aminoalkyl, C₁₋₄ alkylcarbonyl, C₁₋₄ dialkylamino, azido
**Y =** H, halo, alkylamino, dialkylamino, nitrile, hydroxy, C₁₋₆alkylcxycarbonyl, C₁₋₆alkylcarbonyloxy, C₅₋₇ cycloalkyl optionally substituted with up to 4 substituants selected from :
halo, hydroxy, C₁₋₄ alkyl, C₁₋₄ alkoxy, C₁₋₄ hydroxyalkyl, C₁₋₄ alkylamino, amino, C₁₋₄ aminoalkyl, C₁₋₄ alkylcarbonyl, C₁₋₄ dialkylamino, azido, nitrile;
or Y can be : 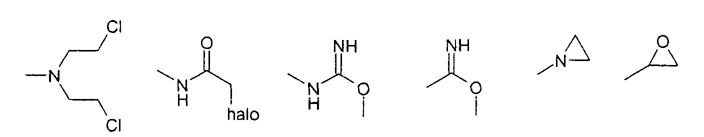 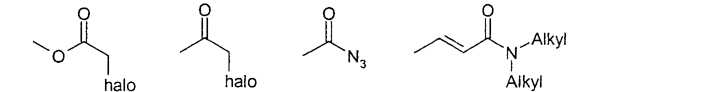
**R2 =** C₇₋₉ cycloalkyl;
C₅₋₈ cycloalkyl substituted with up to 4 substituants;
C₅₋₈cycloalkenyl optionally substituted with up to 4 substituants;
C₅₋₈aliphatic heterocycle optionally substituted with up to 4 substituants;
C₆₋₉bridged cycloalkyl optionally substituted with up to 4 substituants;
C₆₋₉bridged cycloalkenyl optionally substituted with up to 4 substituants;
substituants selected from :
halo, hydroxy, C₁₋₄ alkyl, C₁₋₄ alkoxy, C₁₋₄ hydroxyalkyl, C₁₋₄ alkylamino, amino, C₁₋₄ aminoalkyl, C₁₋₄ alkylcarbonyl, C₁₋₄ dialkylamino, azido, CN; 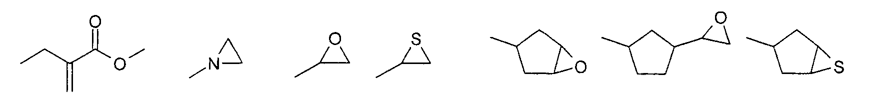 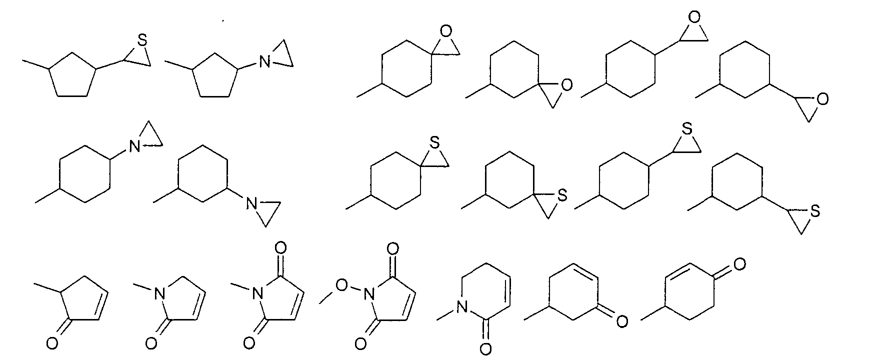 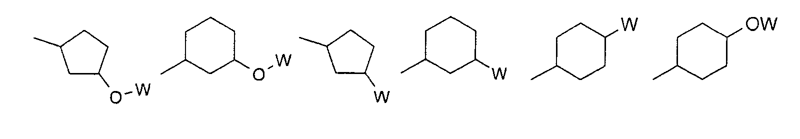 Or R2 can be : 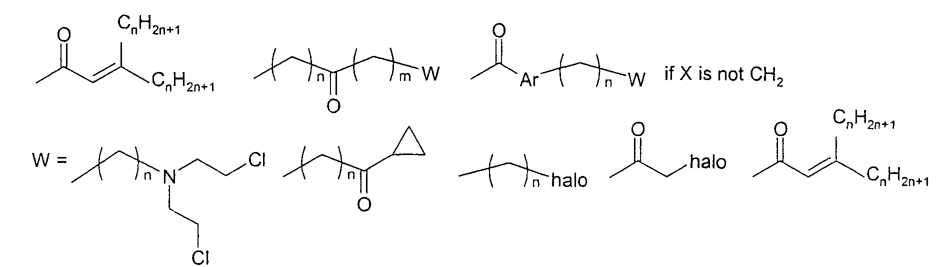 n, m = 0 - 8

2. The compound according to claim 1 further **characterized in that** it has a substituted cycloalkyl group as R2 in position 4 of the pyridinone ring.

3. The compound according to claim 2 further **characterized in that** said substituted cycloalkyl group is a 3,5-dimethylcyclohexyl moiety.

4. The compound according to claim 1 further **characterized in that** it has a C7-9 cycloalkyl group as R2 in position 4 of the pyridinone ring.

5. The compound according to claim 1 selected from the group consisting of compounds which correspond to

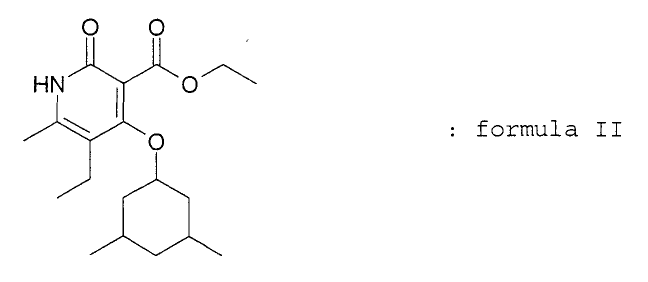

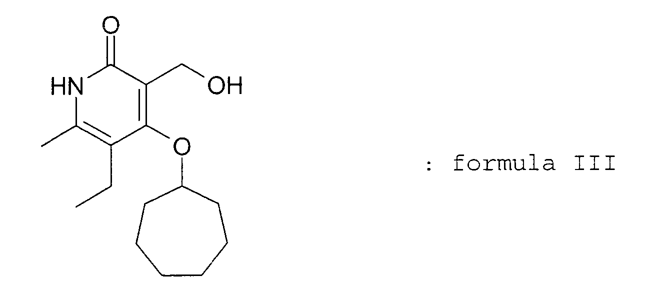

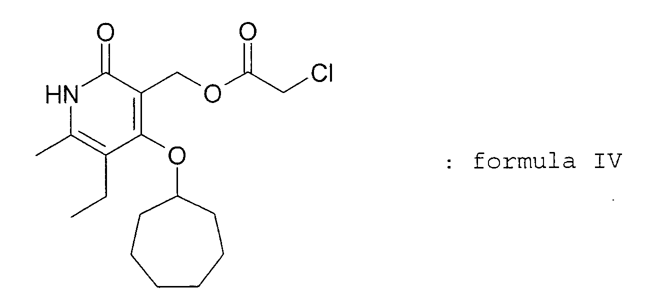

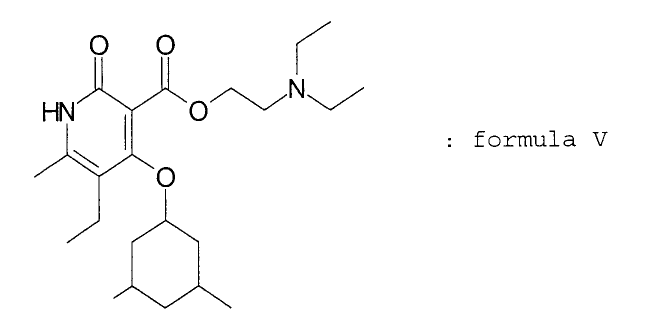

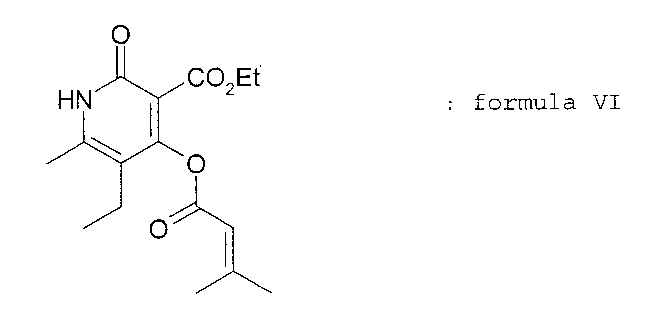

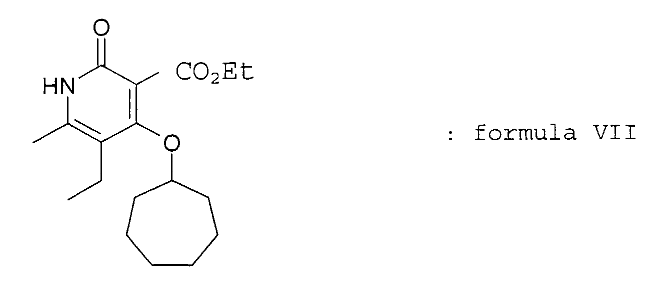

6. A pharmaceutical composition comprising at least one the compounds according to any of claims 1 to 5 and an acceptable carrier and/or diluent.

7. The composition according to claim 6 further comprising another anti-viral agent.

8. The composition according to claim 7, **characterized in that** said anti-viral agent is Nevirapine.

9. Use of the compound or the composition according to any of the preceding claims 1 to 8 for the preparation of a medicament in the treatment and/or the prevention of HIV-1 infections.

10. The use according to the claim 9 for the preparation of a medicament for the treatment and/or prevention of HIV-1 infections by a strain resistant to at least one anti-viral agent.

11. The use of claim 10 wherein said anti-viral agent is Nevirapine.

12. A production process for a compound according to claim 5.
